# FASCICULE DE BREVET EUROPEEN

(11) **EP 1 019 016 B1**
(45) Date de publication et mention de la délivrance du brevet: **07.08.2002**
(21) Numéro de dépôt: 98903103.4
(22) Date de dépôt: 22.01.1998
(51) Int. Cl.: A61K 7/48, A61K 35/78

(54) **COMPLEXE SYNERGIQUE ACTIF ET PRODUIT COSMETIQUE ET/OU PHARMACEUTIQUE COMPRENANT CE COMPLEXE**
AKTIVER SYNERGISTISCHER KOMPLEX UND KOSMETISCHES UND/ODER PHARMAZEUTISCHES PRODUKT DAS DIESEN KOMPLEX ENTHÄLT
ACTIVE SYNERGETIC COMPLEX AND COSMETIC AND/OR PHARMACEUTICAL PRODUCT CONTAINING THIS COMPLEX

(30) Priorité: 03.02.1997 FR 9701325
(43) Date de publication de la demande: 19.07.2000
(73) Titulaire: Cognis France S.A., 31360 Saint Martory (FR)
(72) Inventeur: PAULY, Gilles, F-54280 Seichamps (FR)
(74) Mandataire: Nuss, Pierre
(86) Numéro de dépôt international: FR9800112
(87) Numéro de publication internationale: WO9833475

(56) Documents cités:
- WO-A-86/02833
- WO-A-94/18944
- WO-A-96/11667
- WO-A-96/28008

## Description

La présente invention concerne le domaine de la cosmétologie et de la pharmacologie, notamment préventive et réparatrice, et a pour objet un complexe synergique actif anti-stress, ainsi qu'un produit cosmétique et/ou pharmaceutique intégrant ce dernier.

Au niveau des cellules d'un organisme, en particulier humain, le stress peut être provoqué soit par des agressions externes : chaleur, agressions xénobiotiques (cadmium, arsénite de sodium), irradiation solaire, soit par des privations en glucose, en oxygène, (anoxie) ou encore par des inducteurs endogènes : division cellulaire, différenciation cellulaire.

Tous les organismes vivants, des plus simples (archéobactéries) aux plus évolués (mammifères), présentent la capacité de répondre à un stress par la synthèse d'un ensemble de protéines connues sous la désignation HSP (Heat Shock Protein ou protéines de choc thermique) regroupées en différentes familles en fonction de leur poids moléculaire.

Les agressions précitées entraînent une dénaturation des protéines cellulaires ("protéotoxines") et les protéines de stress (HSP) participent à la "renaturation" de ces protéines ou à leur élimination. Elles permettent ainsi aux cellules de résister au stress.

Durant le stress, l'activité des protéines du type HSP est également déplacée pour protéger le patrimoine génétique (ADN) ainsi que son mode d'expression (nucléole, ribosome, ARN messager).

Au niveau de la peau, on retrouve à l'état physiologique normal l'expression de la plupart des protéines de stress avec, en particulier, une forte expression des protéines de la famille des HSP70, mais aussi de l'HSP27.

L'HSP72 est préférentiellement localisée dans la couche basale et l'HSC70 (HSP constitutive) dans les couches suprabasales.

Au cours de différents stress cutanés, l'expression des HSP au niveau des cellules augmente significativement et ces protéines peuvent être considérées comme de véritables marqueurs cellulaires de stress.

Il a ainsi été montré que pour des kératinocytes en culture, un choc thermique chaud (1 heure à 42° C ou 15 minutes à 47° C) induisait l'expression des HSP72, HSP78, HSP90 et HSP110 et qu'un choc thermique (1 heure à 45°C) induisait l'expression de l'HSP72 et de l'HSP27 dans la peau humaine en culture organotypique.

De même, dans la peau humaine in vivo, il y a induction de l'HSP72 après un choc chaud dans les cellules épidermiques et dermiques et le stress cutané provoqué par irradiation UV-B induit l'apparition de l'HSP72 dans des cultures organotypiques de peau humaine et l'HSP27 dans la peau de souris, in vivo.

De même, il a également été vérifié qu'un traitement UV-A (5 J/cm² à 80 J/cm²) induit l'expression de l'HSP72 dans des cultures cellulaires (lignée cellulaire de fibrosarcome HT1080).

Ainsi, il est notamment bien établi que, au cours d'agressions physiques telles que la chaleur ou l'irradiation UV, les protéines du type HSP sont fortement induites au niveau de l'épiderme cutané. Elles représentent alors un excellent marqueur du degré de stress de la peau dans ces conditions : irradiation ultraviolette accompagnée ou non d'une élévation de température.

Ces marqueurs (HSP) peuvent donc être parfaitement utilisés pour étudier des actifs et/ou des produits cosmétiques et notamment des produits protecteurs solaires (produits solaires ou contre le vieillissement prématuré de la peau).

Il existe actuellement un certain nombre de méthodes pour évaluer l'effet protecteur sur la peau contre les dommages de la lumière solaire, des préparations solaires : mesure du SPF ("Sun Protecting Factor" ou facteur de protection solaire), comptage des SBCs ("Sunburn Cells" ou cellules coups de soleil) dans l'épiderme, mesure de l'effet anti-radicaux libres.

L'évaluation des HSP, et notamment de l'HSP27 et 72, est une méthode originale pour mesurer l'effet photoprotecteur de produits cosmétiques, protecteurs anti-solaire ou anti-vieillissement prématuré de la peau, photo-induit.

On sait, par ailleurs, que le glutathion, un tripeptide composé de trois acides aminés dont la cystéine qui confère à ce tripeptide une fonction thiol (-SH), existe sous forme réduite (GSH) ou oxydé (GS-SG pont disulfure) dans la cellule grâce à un système enzymatique de conversion permettant de passer d'une forme à l'autre et que le glutathion est présent en quantité importante dans la peau.

En comparaison par rapport au derme, il est présent en quantité deux fois plus importante dans l'épiderme, dans la couche des cellules vivantes et dans la couche des cellules mortes ou couche cornée.

Il est bien connu que dans la peau, le glutathion joue un rôle de détoxification des radicaux libres et peroxydes, et également de protection des membranes des cellules vivantes et des protéines structurales de la couche cornée (kératine).

Ce pouvoir de détoxification et de protection s'exerce dans différentes conditions et natures de stress telles que stress oxydatif, stress thermique ou stress par irradiation.

En effet, comme indiqué précédemment, le rayonnement ultraviolet UV-B et aussi UV-A provoque une déplétion du taux de glutathion, dans des cellules en culture in vitro ou in vivo sur souris et il a été démontré que cette déplétion potentialisait la formation des cellules du type cellules coup de soleil dans l'épiderme.

Il a été décrit également que le rayonnement UV-R accroissait la présence de ponts disulfures (S-S) mis en évidence par histochimie dans l'épiderme, en particulier au niveau des cellules coup de soleil apparues après irradiation.

Ces ponts disulfures sont d'ailleurs reconnus comme une étape précédant l'apoptose ou mort cellulaire programmée UV-induite.

La teneur dans l'épiderme en groupements thiol, dont le glutathion, et en ponts disulfures sont donc de bons indicateurs d'une évolution vers un vieillissement et une mort cellulaire accélérés et il apparaît que l'enrichissement de l'épiderme en groupements thiols, et notamment en glutathion, constitue un bon moyen de protection anti-solaire et anti-vieillissement photo-accéléré.

Le document WO 86 02 833 divulgue l'utilisation d'extrait de petit-pois en cosmétologie et dans des compositions cosmétiques.

Afin de lutter contre les effets du rayonnement solaire, différents produits cosmétiques dits "solaires" et "anti-photo-vieillissement" ont été développés et mis au point, leur développement depuis leur début jusqu'à ce jour pouvant être subdivisé en plusieurs étapes dont chacune correspond sensiblement à l'ajout d'un composé actif supplémentaire.

Il a tout d'abord été procédé à l'introduction dans ces produits connus de filtres UV-B (pour éviter les brûlures solaires), puis à l'introduction de filtres UV-A (action sur le photo-vieillissement chronique et la carcinogenèse).

Une troisième étape a consisté en l'ajout de substances anti-oxydantes et anti-radicaux libres (lutte contre la formation des cellules coup de soleil), suivi par l'incorporation de substances prémélanogènes (accélération du bronzage par production d'automélanine) et, enfin, plus récemment, par l'introduction de cytophotoimmunoprotecteurs (préservation du capital immunologique cutané constitué par les cellules de Langerhaus dans l'épiderme).

Indépendamment de l'évolution précitée, qui a abouti à des produits présentant un nombre d'ingrédients croissant sans effets réellement synergique entre eux, les inventeurs de la présente invention ont recherché et développé une nouvelle voie consistant dans le renforcement de l'autodéfense naturelle de la peau, c'est-à-dire en agissant à l'échelon bio-moléculaire de la protection et de la réparation cutanée solaire, par des actions synergiques et simultanées, d'une part, de stimulation de l'autobiosynthèse de glutathion réduit et, d'autre part, d'inhibition de l'apparition de protéines de stress, marqueurs moléculaires d'agressions épidermiques.

Ces recherches ont permis d'aboutir à un complexe synergique présentant en particulier des activités photoprotectrices d'effets délétères biomoléculaires et destiné notamment à être intégré dans une préparation cosmétique et/ou pharmaceutique à usage topique pour la peau et/ou les phanères, comportant au moins un extrait de graines de Pisum Sativum riche en peptides, un extrait de plante de la famille des méliacées, riche en tannins et/ou dérivés coumariniques (et, le cas échéant en triterpenoïdes et/ou en saponosides), et, le cas échéant, au moins un acide aminé sous forme de sel(s) complexe(s).

Ce complexe synergique actif se présente préférentiellement sous forme concentrée et facilement utilisable par les formulateurs cosméticiens.

Conformément à une première caractéristique de l'invention, le complexe synergique comporte entre 0,05 % et 40 % en poids, préférentiellement entre 1 % et 40 % en poids, d'extrait de Pisum Sativum riche en peptides, sous forme déshydratée ou non.

Le procédé de préparation de l'extrait peptidique de graines (pois) de Pisum Sativum peut, à titre d'exemple, consister à broyer les pois en présence d'eau acidulée et d'opérer une agitation à 50° C, puis à centrifuger et à réaliser une ultrafiltration de la suspension obtenue et enfin à filtrer le rétentat et éventuellement à le déshydrater par atomisation ou lyophilisation.

Selon une autre caractéristique de l'invention, le complexe synergique comporte entre 0,005 % et 10 % en poids, préférentiellement entre 0,1 % et 10 % en poids, d'extrait de plante de la famille des méliacées riche en tannins et/ou dérivés coumariniques.

Préférentiellement, l'extrait de plante de la famille des méliacées consiste en un extrait aqueux, alcoolique ou hydroalcoolique déshydraté de Khaya Senegalensis, préférentiellement un extrait de l'écorce de cette plante ou, le cas échéant, un extrait des feuilles ou des graines de cette dernière.

Le procédé de préparation de l'extrait d'écorce de Khaya Senegalensis peut consister, par exemple, à broyer l'écorce de cette plante, puis à la mettre en suspension à 20 % dans de l'eau distillée, en agitant pendant deux heures à 90° C ou en laissant en macération à température ambiante pendant deux jours, et enfin à opérer une séparation, une filtration, suivie éventuellement d'une déshydratation (atomisation ou lyophilisation).

L'extraction de la fraction active (comprenant notamment des tannins et/ou dérivés coumariniques) pourra également être opérée dans les mêmes conditions en utilisant comme solvant l'éthanol ou un mélange eau/éthanol.

Conformément à une autre caractéristique de l'invention, l'acide aminé ou les acides aminés présent(s) appartien(en)t au groupe formé par l'histidine, l'arginine et la tyrosine, ledit ou lesdits acide(s) aminé(s) étant présent(s) sous forme de sels d'acide succinique et/ou d'acide aspartique.

De manière avantageuse, les acides aminés sont présents sous forme pure, sous forme de sels d'acide succinique et/ou d'acide aspartique et/ou sous forme de mélanges du type (sel d'acide aminé ou acide aminé / acide succinique ou acide aspartique), représentant ensemble entre 0,05 % et 40 % en poids, préférentiellement entre 0,1 % et 40 % en poids, du complexe synergique actif.

Outre les composants précités, le complexe synergique selon l'invention peut également comporter (i) entre 0,05 % et 5 % en poids d'un extrait hydrosoluble de cellules de levure, du type Saccharomyces Cerevisiae, (ii) entre 0,05 % et 10 % en poids d'oses, de polyosides et/ou de polysaccharides, notamment de saccharose et/ou de glycogène, et/ou (iii) entre 0,01 % et 10 % en poids de vitamine(s) du groupe B, telles que notamment la pyridoxine et/ou la niacinamide.

Compte tenu des différentes caractéristiques mentionnées ci-dessus, une formulation pondérale type d'un complexe synergique actif selon l'invention peut se présenter comme suit:

| | |
|---|---|
| 1) extrait peptidique de graines de Pisum Sativum (pois) | 1 à 40 % |
| 2) extrait aqueux ou hydroalcoolique d'écorce de Khaya Senegalensis | 0,1 à 10 % |
| 3) acides aminés (histidine, arginine et/ou tyrosine) sous forme de sels d'acide(s) succinique et/ou aspartique | 0,1 à 40 % |
| 4) extrait hydrosoluble de levure | 0,05 à 5 % |
| 5) vitamine B (pyridoxine et/ou niacinamide) | 0,01 à 10 % |
| 6) polyoside (glycogène) | 0,05 à 10 % |
| 7) support et/ou solvant | qsp 100 % |

Le complexe synergique précité peut être conditionné sous des formes galéniques variées telles qu'un liquide, un soluté, une pâte, une poudre, des liposomes, des microsphères, des microcapsules, des nanoparticules ou analogues.

Les exemples 1 à 7 ci-après illustrent, de manière non limitative, différentes formulations pondérales possibles (exprimées en %) pour le complexe synergique actif selon l'invention.

### Exemple 1 :

| | |
|---|---|
| Extrait peptidique de Pisum Sativum atomisé | 30,00 |
| Extrait aqueux d'écorce de Khaya Senegalensis lyophilisé | 2,00 |
| Sorbitol | qsp 100,00 |

### Exemple 2 :

| | |
|---|---|
| Extrait peptidique de Pisum Sativum | 30,00 |
| Extrait aqueux d'écorce de K. Senegalensis lyophilisé | 2,00 |
| Acide succinique, sel d'Histidine | 2,00 |
| Acide succinique, sel d'Arginine | 3,00 |
| Aspartate d'Arginine | 2,00 |
| Tyrosine | 1,00 |
| Extrait hydrosoluble de Saccharomyces Cerevisiae | 2,00 |
| Sorbitol | qsp 100,00 |

### Exemple 3 :

| | |
|---|---|
| Extrait peptidique de Pisum Sativum | 30,00 |
| Extrait hydroalcoolique d'écorce de K. Senegalensis | 0,50 |
| Acide succinique, sel d'Histidine | 1,00 |
| Acide succinique, sel d'Arginine | 5,00 |
| Aspartate d'Arginine | 0,50 |
| Tyrosine | 1,00 |
| Extrait hydrosoluble de Saccharomyces Cerevisiae | 2,00 |
| Mannitol | qsp 100,00 |

### Exemple 4 :

| | |
|---|---|
| Extrait peptidique de Pisum Sativum | 15,00 |
| Extrait d'écorce de K. Senegalensis | 5,00 |
| Succinate d'Histidine et d'Arginine | 10,00 |
| Tyrosine | 1,00 |
| Glycogène | 5,00 |
| Sorbitol | qsp 100,00 |

### Exemple 5 :

| | |
|---|---|
| Extrait peptidique de Pisum Sativum atomisé | 10,00 |
| Extrait d'écorce de K. Senegalensis (lyophilisat) | 5,00 |
| Acide succinique, sel d'Histidine et d'Arginine | 20,00 |
| Aspartate d'Arginine | 2,00 |
| Tyrosine | 1,00 |
| Pyridoxine, CIH | 3,00 |
| Sorbitol | qsp 100,00 |

### Exemple 6:

| | |
|---|---|
| Extrait peptidique de Pisum Sativum | 20,00 |
| Extrait hydroalcoolique d'écorce de K. Senegalensis (lyophilisat) | 0,50 |
| Acide succinique, sel d'Histidine et d'Arginine | 10,00 |
| Aspartate d'Arginine | 5,00 |
| Tyrosine | 1,50 |
| Extrait hydrosoluble de Saccharomyces Cerevisiae | 3,00 |
| Mannitol | qsp 100,00 |

### Exemple 7 :

| | |
|---|---|
| Extrait peptidique de Pisum Sativum (atomisat) | 10,00 |
| Extrait aqueux d'écorce de K. Senegalensis (lyophilisât) | 1,00 |
| Acide succinique / Histidine (25/75) | 4,50 |
| Arginine / Acide Aspartique (75/25) | 4,75 |
| Tyrosine | 1,00 |
| Extrait hydrosoluble de Saccharomyces Cerevisiae (lyophilisat) | 1,50 |
| Pyridoxine, ClH | 1,00 |
| Mannitol | qsp 100,00 |

La présente invention a également pour objet l'utilisation d'un complexe synergique tel que décrit ci-dessus en tant que composé actif pour la préparation d'une composition ou d'un produit cosmétique et/ou pharmaceutique à usage topique pour la peau et/ou les phanères.

Ledit complexe synergique peut être utilisé en tant qu'agent actif anti-stress cutané, démontrée par une réduction de l'expression des protéines de stress connues sous la désignation HSP, notamment HSP27, induite au niveau de l'épiderme suite à des stress du type irradiations solaires UV-A, UV-B et visibles, et/ou à un échauffement de la peau.

Il empêche ainsi ou inhibe l'apparition de protéines de stress induites par des irradiations solaires et réduit, par conséquent, les effets néfastes du type inflammation locale et radicaux libres générés par ces irradiations.

Ledit complexe synergique peut également être utilisé, d'une part, en tant qu'agent stimulateur de l'autosynthèse de glutathion réduit, par les cellules cutanées ou capillaires et/ou, d'autre part, en tant qu'agent s'opposant à l'apparition des ponts disulfures induits par les irradiations solaires aiguës ou résultant du vieillissement cutané chronique photo-induit.

Préférentiellement, il est intégré dans un produit du type photoprotecteur solaire ou après-soleil, seul ou en association complémentaire avec des filtres solaires, ou dans un produit de soin cutané de jour, à action préventive et/ou réparatrice des effets du vieillissement et/ou anti-pollution (en association ou non avec des filtres solaires).

Enfin, la présente invention a également pour objet un produit cosmétique et/ou pharmaceutique pour la peau et/ou les phanères (liquide, lotion, émulsion, crème ou analogue), présentant notamment une activité cytophotoprotectrice moléculaire, caractérisé en ce qu'il comporte entre 0,01 % et 90 % en poids, avantageusement entre 0,05 et 20 % en poids, d'un complexe synergique actif tel que décrit ci-dessus, éventuellement associé à des filtres solaires.

Le produit cosmétique et/ou pharmaceutique précité comporte, de manière préférentielle, entre 1 et 10 % en poids du complexe synergique actif selon l'invention et on observe, suite à des applications topiques externes dudit produit, un effet protecteur, photo-protecteur et anti-inflammatoire local.

A titre d'exemples non limitatifs de réalisations pratiques de compositions selon l'invention, on décrira ci-après différent(e)s produits ou préparations cosmétiques comprenant le complexe synergique actif précité; l'exemple 8 qui ne contient pas le complexe revendiqué, illustre une composition conforme à l'art antérieur.

### Exemple 1:

Un produit cosmétique sous forme d'émulsion anti-stress pour le visage conforme à l'invention pourra, par exemple, présenter une composition pondérale, constituée à partir des fractions A. B et C suivantes, telle qu'indiquée ci-après.

### Fraction A :

- Stéarate de glycérol et sétarate de PEG 100 1,500
- Stéarate de glycérol et CETETH-20 1,500
- Alcool cétylique 1,000
- Triglycéride caprylique / caprique 5,000
- Isononanoate cétostéarylique 4,000
- Octyldodécanol 3,000
- Diméthicone 0,500
- Elestab 4121 (Laboratoires Sérobiologiques) 0,300

### Fraction B :

- Eau 73,550
- Elestab 4112 (Laboratoires Sérobiologiques) 0,400
- Glycérine 5,000
- Gomme xanthane 0,500
- Complexe synergique actif (exemple 6) 5,000

### Fraction C :

- Polyacrylamide et Isoparaffine et Laureth-7 0,750

Le procédé de préparation et de fabrication de l'émulsion pour le visage précitée consiste essentiellement à préparer la fraction B (dissolution de l'Elestab 4112 dans l'eau et ajout de la glycérine à 75° C, puis ajout de la gomme xanthane et dissolution du complexe synergique), à préparer la fraction A à 75° C et à la verser dans la fraction B à 75° C, sous agitation turbine, à ajouter ensuite la fraction C à 60° C, sous agitation turbine, à laisser refroidir et à mettre en oeuvre une agitation planétaire à partir de 50° C, jusqu'au retour à température ambiante.

### Exemple 2 :

Un produit cosmétique sous forme de crème protectrice et anti-âge pour le cou conforme à l'invention pourra, par exemple, présenter une composition pondérale, constituée à partir des fractions A et B suivantes, telle qu'indiquée ci-après.

### Fraction A :

- Palmitate de sorbitol 3,500
- Stéarate de glycérol 1,500
- Alcool cétylique 2,500
- Isononanoate cétostéarylique 7,000
- Octyldodécanol 2,500
- Huile de paraffine 3,000
- Diméthicone 2,000
- Elestab 4121 (Laboratoires Sérobiologiques) 0,300

### Fraction B :

- Eau 69,100
- Elestab 4112 (Laboratoires Sérobiologiques) 0,400
- Glycérine 4,000
- Sulfate cétostéarylique de Sodium 1,200
- Complexe synergique actif (exemple 3) 2,000

Le procédé de préparation et de fabrication de la crème précitée consiste essentiellement à préparer la fraction B à 75° C, à y verser sous agitation turbine la fraction A à 80° C, à laisser refroidir et à maintenir l'agitation turbine, jusqu'à 50° C, et à poursuivre le refroidissement au planétaire, jusqu'à retour à température ambiante.

### Exemple 3 :

Un produit cosmétique sous forme de crème solaire peut par exemple, présenter une composition pondérale, constituée à partir des fractions A et B suivantes, telle qu'indiquée ci-après.

### Fraction A :

- Alcool cétylique 5,000
- Triglycéride caprylique / caprique 6,100
- Huile de paraffine 3,750
- Lanoline 1,000
- Stéarate de glycérol 2,000
- Diméthicone 0,250
- Octyldodécanol 4,500
- Octyl Stéarate 8,150
- Octyl Méthoxycinnamate 6,800
- Butyl Méthoxybenzoyl Méthane 2,000
- Elestab 4121 (Laboratoires Sérobiologiques) 0,300

### Fraction B :

- Eau 53,750
- Elestab 4112 (Laboratoires Sérobiologiques) 0,400
- Glycérine 3,000
- Phosphate cétylique de potassium 3,000

Le procédé de préparation et de fabrication de la crème solaire précitée consiste essentiellement à préparer la fraction A et la fraction B à 75° C, à ensuite verser la fraction A, sous agitation turbine, dans la fraction B à 75° C et à refroidir le mélange jusqu'à température ambiante, sous agitation planétaire.

### Exemple 4 :

Un produit cosmétique sous forme de crème solaire conforme à l'invention pourra, par exemple, présenter une composition pondérale, constituée à partir des fractions A et B suivantes, telle qu'indiquée ci-après.

### Fraction A :

- Alcool cétylique 5,000
- Triglycéride caprylique / caprique 6,100
- Huile de paraffine 3,750
- Lanoline 1,000
- Stéarate de glycérol 2,000
- Diméthicone 0,250
- Octyldodécanol 4,500
- Octyl Stéarate 8,150
- Octyl Méthoxycinnamate 6,800
- Butyl Méthoxybenzoyl Méthane 2,000
- Elestab 4121 (Laboratoires Sérobiologiques) 0,300

### Fraction B :

- Eau 50,750
- Elestab 4112 (Laboratoires Sérobiologiques) 0,400
- Glycérine 3,000
- Phosphate cétylique de potassium 3,000
- Complexe synergique (exemple 7) 3,000

Le procédé de préparation et de fabrication de la crème solaire précitée consiste essentiellement à préparer la fraction A à 80° C et la fraction B à 75° C. puis à verser la fraction A à 80° C sous agitation turbine dans la fraction B à 75° C et à refroidir le mélange jusqu'à température ambiante, sous agitation planétaire.

Afin d'illustrer et de démontrer les avantages des agents complexes selon les exemples précités (leurs propriétés originales et leur intérêt et spécificité par rapport aux filtres solaires UV-B et UV-A), les tests d'objectivation suivants (A, B, C, D et E) ont été réalisés avec le complexe synergique actif correspondant à l'exemple 7 décrit précédemment.

Les résultats de ces tests ont été représentés de manière graphique sur les figures 1 à 8 des dessins annexés, dans lesquels :
- la figure 1 est une représentation graphique du taux de glutathion des fibroblastes humains MRC5 en test de survie (test d'activité A) [Test de Student - (∗∗) indique que p (risque d'erreur) <0,01] ;
- la figure 2 est une représentation graphique du taux de PGE2 (prostaglandine E2) de kératinocytes humains NCTC2544 sous rayonnement UV-B (30 mJ/cm²) - test d'activité C [Test t de Student - (∗) indique que p < 0,05 et (∗∗) indique que p < 0,01] ;
- la figure 3 est une représentation graphique du taux de LDH (lactate déshydrogénase relargué dans le limieu de culture surnageant) de kératinocytes humains NCTC2544 sous rayonnement UV-B (30 mJ/cm²) - test d'activité C / traitement simultané [Test t de Student - (∗) indique que p < 0,05 et (∗∗) indique que p<0,001] ;
- la figure 4 est une représentation graphique du pourcentage d'inhibition de la lipoxygénase par dosage des anions superoxydes formés intubo - test d'activité C ;
- la figure 5 est une représentation graphique du taux de MDA (malonaldialdéhyde) de fibroblastes MRC5 sous rayonnement UV-A (15 J/cm²) - test d'activité D / traitement préventif [(∗) indique que p < 0,05] ;
- la figure 6 est une représentation graphique du taux de GSH (glutathion réduit) de fibroblastes MRC5 sous rayonnement UV-A (15 J/cm²) - test d'activité D / traitement préventif [Test t de Student - (∗) indique que p < 0,05] ;
- la figure 7 est une représentation graphique montrant l'effet protecteur sur peau humaine, ex-vivo, en culture organotypique, avant une irradiation par un simulateur solaire, vis à vis de l'induction de la protéine de stress HSP27 visualisée par immunohistochimie, au niveau des couches vivantes de l'épiderme - test d'activité E [U de Mann et Whitney - (∗∗) indique que p < 0,001 et (NS) veut dire "non significatif"] ; et,
- la figure 8 est une représentation graphique montrant l'effet protecteur sur peau humaine, ex-vivo, en culture organotypique, avant une irradiation par un simulateur solaire, vis à vis de l'induction des ponts S-S visualisés histochimie, au niveau des couches vivantes de l'épiderme - test d'activité E [U de Mann et Whitney - (∗∗) indique que p < 0,001 et (NS) veut dire "non significatif"].

A) Activité nutritive et eutrophique sur fibroblastes humains en survie (in vitro).

Les capacités eutrophiques des complexes synergiques actifs selon l'invention ont été évaluées par un test de survie sur fibroblastes humains MRC5. Les doses à tester ont été déterminées préalablement par un test de toxicité sur fibroblastes MRC5.

Pour le test de survie, le produit a été dissous dans le milieu de culture standard DMEM et mis en contact des MRC5 trois jours après l'ensemencement. Puis au bout de sept jours d'incubation à 37° C (CO₂ = 5 %), la survie des cellules a été évaluée par dosage du glutathion (GSH) intracellulaire par une sonde fluorescente (OPTH), selon la méthode de HISSIN P.J. et de HILF R..

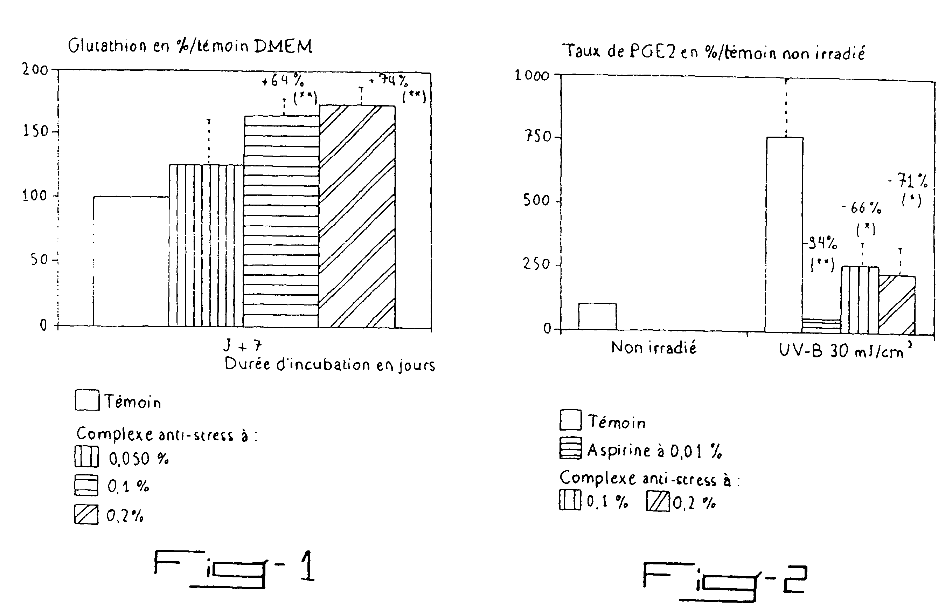

Le complexe anti-stress a amélioré significativement le taux de GSH des MRC5 en survie : + 64 % et + 74 % à J + 7 pour 0,1 et 0,2 % (voir figure 1).

B) Activité anti-radicaux libres (tests in tubo)

Les capacités anti-radicaux libres ont été évaluées par une batterie de tests recouvrant les formes radicalaires initiales ainsi que les formes réactives de l'oxygène induites.
1) Test anti-radicaux libres au DPPH° : ce test a évalué les capacités du complexe synergique à stabiliser un radical libre coloré, le diphénylpycrylhydrazyl (DPPH°) en son leucodérivé stable.
2) Tests anti-radicaux hydroxyles (OH°) par la réaction de Fenton : ces tests ont évalué les capacités à scavenger les OH° formés par le fer avec H₂O₂ et révélés par l'acide salicylique (réaction colorée) ou par le désoxyribose (le désoxyribose est un composé essentiel de l'ADN, et ses produits d'oxydation par OH° sont révélés par l'acide thiobarbiturique). De plus, un essai est réalisé sans EDTA pour déterminer les capacités à capter le fer (effet ferriprive).
3) Tests anti-anions superoxydes (O₂⁻•): O₂⁻• est produit durant les stress oxydatifs par induction de la xanthine oxydase qui va dégrader les coenzymes NAD(P) et l'hypoxanthine en excès par arrêt du métabolisme énergétique dans les tissus (O₂⁻• se dismute spontanément ou en présence de superoxyde dismutase en H₂O₂).

Ces tests biochimiques sont réalisés par mélange d'hypoxanthine et xanthine oxydase et révélation des O₂⁻• par le luminol ou révélation des O₂⁻• et H₂O₂ par le luminol + microperoxydase.

Les résultats (tableau ci-dessous) ont montré que le complexe synergique actif selon l'invention présentait un large spectre d'activité anti-radicaux libres, couvrant aussi bien les formes radicalaires initiales (DPPH°) que les formes réactives de l'oxygène induites (OH°, O₂⁻• et H₂O₂) ainsi qu'une activité ferriprive démontrée par la réaction de Fenton sur le désoxyribose sans EDTA (CI50 = 0,23 %) (CI50 = concentration de produit qui réduit de 50 % l'intensité de la réaction enzymatique - p/v = poids / volume).

| **Tests** | **CI50 (en % p/v)** |
|---|---|
| **Tests chimiques :** | |
| DPPH° | CI50 = 0,36 % |
| Réaction de Fenton : Acide salicylique | CI50 = 0,12 % |
| Désoxyribose avec EDTA | CI50 = 0,14 % |
| sans EDTA | CI50 = 0,23 % |

| **Tests biochimiques :** | |
|---|---|
| Méthode au luminol | CI50 = 0,04 % |
| Méthode au luminol + microperoxydase | CI50 = 0,70 % |

C) Activité cytophotoprotectrice anti-inflammatoire vis-à-vis des UV-B (test in vitro)

Les UV-B déclenchent un processus inflammatoire (érythème, oedème) par activation d'une enzyme, la phospholipase A2, qui libère de l'acide arachidonique (acide gras insaturé) à partir des membranes biologiques.

L'acide arachidonique est le précurseur des médiateurs de l'inflammation tels que les prostaglandines et les leucotriènes.

Les prostaglandines (dont la PGE2) sont formées par l'action de cyclooxygénases, puis sont sécrétées hors de la cellule et agissent par l'intermédiaire de récepteurs spécifiques (oedème, érythème, immunosuppression).

Les leucotriènes sont formés par les lipoxygénases et agissent (comme les prostaglandines) sur des récepteurs spécifiques. Ainsi, le leucotriène LTB4 attire et active les polynucléaires neutrophiles qui vont libérer des radicaux libres et des protéases détruisant les tissus.

L'activité de la lipoxygénase produit également des anions superoxydes qui amplifient le stress oxydatif induit par les prostaglandines et les leucotriènes.

L'effet anti-PGE2 du complexe synergique actif selon l'invention a été évalué sur une culture de kératinocytes humains, arrivés à saturation in vitro.

Le milieu de croissance est remplacé par une solution saline glucosée (contenant les diverses concentrations du complexe synergique) puis les cultures sont irradiées (30 mJ/cm² : tubes fluorescents UV-B) et incubées de nouveau une nuit à 37° C, CO₂ = 5 %.

Le nombre de cellules a été quantifié après trypsination par un compteur de particules. Sur le milieu surnageant, le taux de PGE2 a été évalué par un test ELISA tandis que le taux de LDH (lactate déshydrogénase) a été dosé par réaction enzymatique (DO à 340 nm).

L'inhibition de la lipoxygénase a été déterminée in tubo par quantification des anions superoxydes (par luminescence) produits par cette enzyme en présence d'acides gras insaturés.

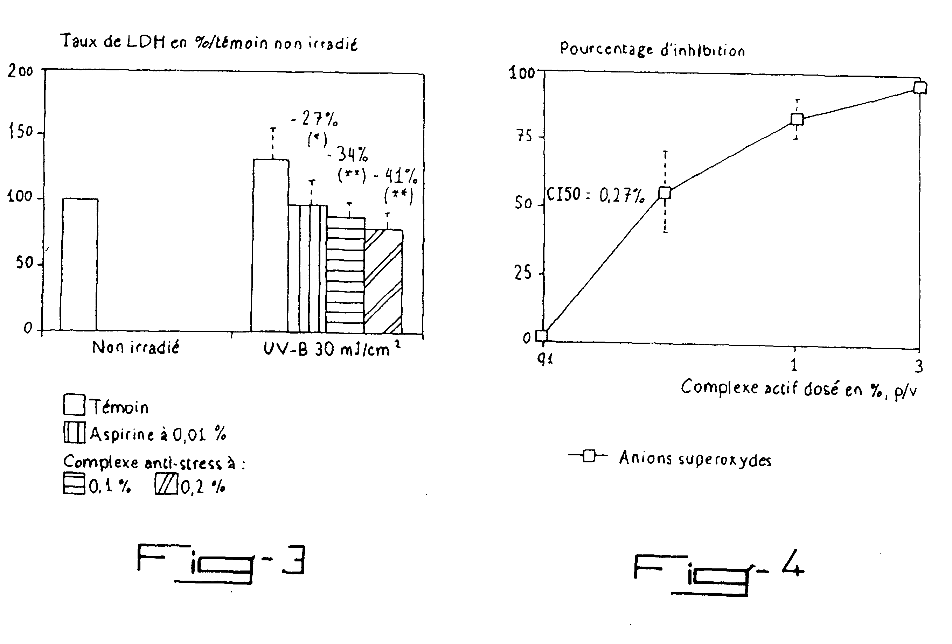

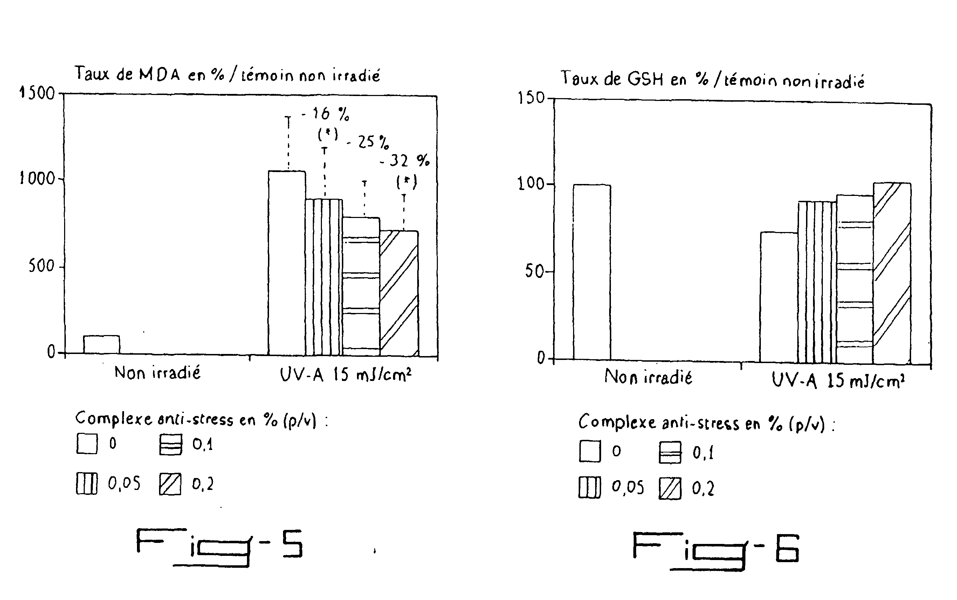

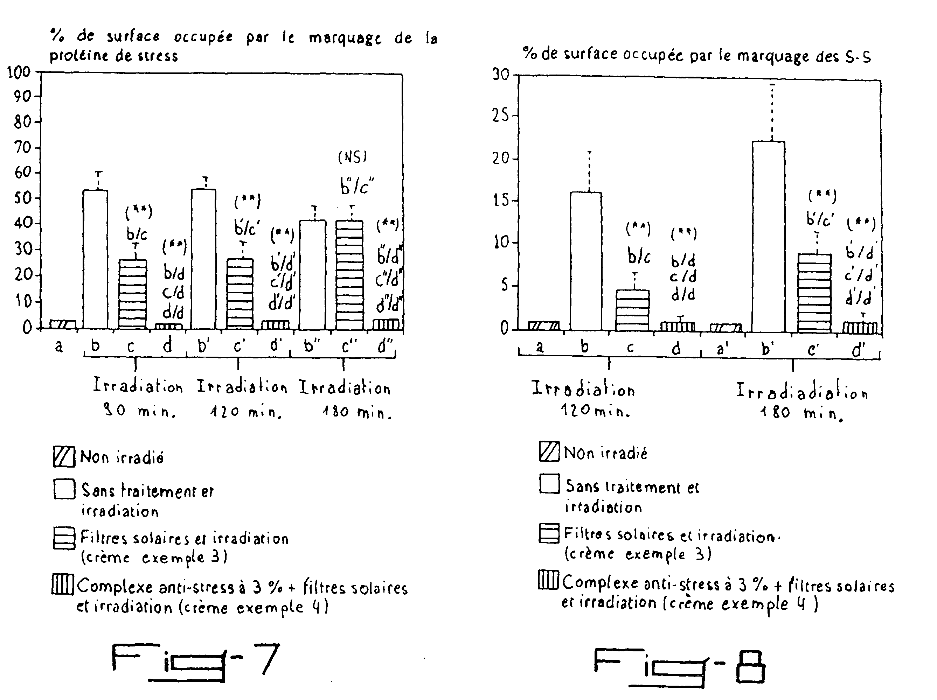

Le complexe synergique selon l'invention (0,2 %, p/v) (voir figures 2, 3 et 4) a réduit fortement le taux de PGE2 (- 71 %) et de LDH (- 41 %) relargués après l'irradiation UV-B.

Le complexe synergique présente une CI50 de 0,27 % (p/v) vis-à-vis des anions superoxydes induits par la lipoxygénase.

Comparativement à l'aspirine, le complexe anti-stress est moins actif vis-à-vis des PGE2 mais, par contre, il est plus actif vis-à-vis de la LDH (qui caractérise la souffrance membranaire) et vis-à-vis de la lipoxygénase.

Le complexe synergique selon l'invention présente des capacités anti-inflammatoires suite à irradiation UV-R non seulement par inhibition de la cyclooxygénase et par cytophotoprotection des membranes biologiques, mais aussi par capture des anions superoxydes produits par l'activité des lipoxygénases sur les acides gras insaturés.

D) Activités cytophotoprotectrice vis-à-vis des UV-A et stimulante de l'auto-synthèse du glutathion (test in vitro sur fibroblastes humains MRC5)

Des fibroblastes MRC5 ont été mis en culture dans un milieu de croissance jusqu'à saturation du tapis cellulaire.

Puis, le milieu de croissance a été remplacé par un milieu standard contenant le complexe synergique aux diverses doses à tester.

Après incubation de 48 heures à 37° C, les différents milieux ont été remplacés par une solution saline, puis les MRC5 ont été irradiés (par des tubes UV-A).

Dès la fin de l'irradiation, la solution saline a été prélevée, pour le dosage du malonaldialdéhyde (MDA) par réaction avec de l'acide thiobarbiturique à chaud (fluorescence à 560 nm). Les cellules MRC5 ont été récupérées pour le dosage des protéines (méthode de Bradford) et du glutathion (GSH) par une sonde fluorescente (le MDA est un produit de dégradation des lipides insaturés qui composent les membranes biologiques et il provoque des pontages qui inhibent les enzymes et forment les lipofuscines (taches de vieillesse) et serait mutagène).

Les résultats (voir figures 5 et 6) montrent que les UV-A (15 J/cm²) ont fortement induit la sécrétion de MDA (x 10) et ont fait chuter de 25 % environ le taux de GSH dans les MRC5.

Le complexe synergique actif selon l'invention a réduit la lipoperoxydation induite par les UV-A : -32 % pour la dose de 0,2 % (p/v).

Le complexe synergique a inhibé la destruction du GSH par les UV-A : + 41 % pour la dose de 0,2 % (p/v).

Le complexe synergique a donc réduit la lipoperoxydation des fibroblastes, induite par les UV-A, notamment par augmentation du taux du glutathion réduit.

E) Activité anti-protéines de stress et anti-ponts disulfures (test sur peau humaine ex-vivo)

Le but de cette dernière étude était de tester l'efficacité antisolaire du complexe synergique actif à 3 % en association avec un mélange de filtres UV-A + UV-B, comparativement à ces filtres solaires seuls, lors d'irradiations cutanées, ex vivo, par un simulateur solaire.

Afin d'apprécier l'efficacité antisolaire du complexe anti-stress, l'irradiation de peaux humaines en culture organotypique (ex vivo), a été réalisée avec ou sans traitement topique par les produits solaires à tester, dont la composition a été précisée aux exemples 3 et 4 précités.

L'activité des produits a ensuite été évaluée par immunohistochimie + analyse d'images sur deux marqueurs du stress solaire : l'induction de la protéine de stress HSP27 et l'apparition des ponts disulfures (S-S) dans les couches vivantes de l'épiderme.

Les résultats de l'expérimentation (voir figures 7 et 8) ont permis de constater que :
- dans l'épiderme de la peau humaine normale non stressée, le protéine de stress HSP27 n'est pas exprimée et les ponts disulfures ne sont présents qu'au niveau du stratum corneum,
- pour les trois temps d'irradiation étudiés, on observe une induction très importante de la protéine de stress,
- il y a apparition de nombreux ponts disulfures au niveau des couches vivantes de l'épiderme pour les deux temps d'irradiation les plus élevés,
- l'application des crèmes contenant les filtres solaires, avant l'irradiation, limite dans la plupart des cas partiellement l'induction de la protéine de stress, et, dans tous les cas, l'apparition des ponts S-S,
- par contre, l'application de la crème contenant le complexe synergique actif, avant l'irradiation, évite presque totalement l'expression de la protéine de stress HSP27 et l'apparition des ponts S-S dans les couches vivantes de l'épiderme humain.

Bien entendu, l'invention n'est pas limitée au mode de réalisation décrit et représenté aux dessins annexés. Des modifications restent possibles, notamment du point de vue de la constitution des divers éléments ou par substitution d'équivalents techniques, sans sortir pour autant du domaine de protection de l'invention.

## Revendications

1. Complexe synergique destiné notamment à être intégré dans une préparation cosmétique et/ou pharmaceutique à usage topique pour la peau et/ou les phanères, **caractérisé en ce qu'**il comporte au moins un extrait de graines de Pisum Sativum riche en peptides, un extrait de plante de la famille des méliacées, riche en tannins et/ou dérivés coumariniques et, le cas échéant, au moins un acide aminé sous forme de sel(s) complexe(s).

2. Complexe synergique selon la revendication 1, **caractérisé en ce qu'**il comporte entre 0,05 % et 40 % en poids, préférentiellement entre 1 % et 40 % en poids, d'extrait de Pisum Sativum riche en peptides, sous forme déshydratée ou non.

3. Complexe synergique selon l'une quelconque des revendications 1 et 2, **caractérisé en ce qu'**il comporte entre 0,005 % et 10 % en poids, préférentiellement entre 0,1 % et 10 % en poids, d'extrait de plante de la famille des méliacées riche en tannins et/ou dérivés coumariniques.

4. Complexe synergique selon la revendication 3, **caractérisé en ce que** l'extrait de plante de la famille des méliacées consiste en un extrait aqueux, alcoolique ou hydroalcoolique déshydraté de Khaya Senegalensis, préférentiellement un extrait de l'écorce de cette plante.

5. Complexe synergique selon l'une quelconque des revendications 1 à 4, **caractérisé en ce que** l'acide aminé ou les acides aminés présent(s) appartien(en)t au groupe formé par l'histidine, l'arginine et la tyrosine.

6. Complexe synergique selon la revendication 5, **caractérisé en ce que** les acides aminés sont présents sous forme de sels d'acide succinique et/ou d'acide aspartique.

7. Complexe synergique selon la revendication 5, **caractérisé en ce que** les acides aminés sont présents sous forme pure, sous forme de sels d'acide succinique et/ou d'acide aspartique et/ou sous forme de mélanges du type (sel d'acide aminé ou acide aminé / acide succinique ou acide aspartique), représentant ensemble entre 0,05 % et 40 % en poids, préférentiellement entre 0,1 % et 40 % en poids, du complexe synergique actif.

8. Complexe synergique selon l'une quelconque des revendications 1 à 7, **caractérisé en ce qu'**il comporte également entre 0,05 % et 5 % en poids d'un extrait hydrosoluble de cellules de levure, du type Saccharomyces Cerevisiae.

9. Complexe synergique selon l'une quelconque des revendications 1 à 8, **caractérisé en ce qu'**il comporte également entre 0,05 % et 10 % en poids d'oses, de polyosides et/ou de polysaccharides, notamment de saccharose et/ou de glycogène.

10. Complexe synergique selon l'une quelconque des revendications 1 à 9, **caractérisé en ce qu'**il comporte également entre 0,01 % et 10 % en poids de vitamine(s) du groupe B, telles que notamment la pyridoxine et/ou la niacinamide.

11. Utilisation d'un complexe synergique selon l'une quelconque des revendications 1 à 10 en tant que composé actif pour la préparation d'une composition ou d'un produit cosmétique et/ou pharmaceutique à usage topique pour la peau et/ou les phanères.

12. Utilisation selon la revendication 11, **caractérisée en ce que** le complexe synergique est utilisé en tant qu'agent actif anti-stress cutané, démontrée par une réduction de l'expression des protéines de stress connues sous la désignation HSP, notamment HSP27.

13. Utilisation selon l'une quelconque des revendications 11 et 12, **caractérisée en ce que** le complexe synergique actif est utilisé en tant qu'agent stimulateur de l'autosynthèse de glutathion réduit, par les cellules cutanées ou capillaires.

14. Utilisation selon l'une quelconque des revendications 11 à 13, **caractérisée en ce que** le complexe synergique actif est utilisé en tant qu'agent s'opposant à l'apparition des ponts disulfures induits par les irradiations solaires aiguës ou résultant du vieillissement cutané chronique photo-induit.

15. Utilisation selon l'une quelconque des revendications 11 à 14, **caractérisée en ce que** le complexe synergique actif est intégré dans un produit du type photoprotecteur solaire ou dans un produit de soin cutané de jour, à action préventive et/ou réparatrice des effets du vieillissement.

16. Produit cosmétique pour la peau et/ou les phanères, présentant notamment une activité cytophotoprotectrice moléculaire, **caractérisé en ce qu'**il comporte entre 0,01 % et 90 % en poids d'un complexe synergique selon l'une quelconque des revendications 1 à 10.

17. Produit pharmaceutique pour la peau et/ou les phanères, présentant notamment une activité cytophotoprotectrice moléculaire, **caractérisé en ce qu'**il comporte entre 0,01 % et 90 % en poids d'un complexe synergique selon l'une quelconque des revendications 1 à 10.

## Patentansprüche

1. Synergistischer Komplex, insbesondere zur Einarbeitung in ein Kosmetik- oder Arzneimittelpräparat zur topischen Verwendung für die Haut und/oder die Epithelanhanggebilde, **dadurch gekennzeichnet, daß** er mindestens einen peptidhaltigen Extrakt von Pisum sativum-Körnern, einen tannin- und/oder cumarinderivathaltigen Pflanzenextrakt der Familie der Meliaceen und gegebenenfalls mindestens eine Aminosäure in Form des komplexen Salzes bzw. komplexer Salze enthält.

2. Synergistischer Komplex nach Anspruch 1, **dadurch gekennzeichnet, daß** er 0,05 Gew.-% bis 40 Gew.-%, vorzugsweise 1 Gew.-% bis 40 Gew.-% peptidhaltigen Extrakt von Pisum sativum in entwässerter oder nicht entwässerter Form enthält.

3. Synergistischer Komplex nach Anspruch 1 oder 2, **dadurch gekennzeichnet, daß** er 0,005 Gew.-% bis 10 Gew.-%, vorzugsweise 0,1 Gew.-% bis 10 Gew.-%, tannin- und/oder cumarinderivathaltigen Pflanzenextrakt der Familie der Meliaceen enthält.

4. Synergistischer Komplex nach Anspruch 3, **dadurch gekennzeichnet, daß** der Pflanzenextrakt der Familie der Meliaceen aus einem entwässerten wäßrigen, alkoholischen oder wäßrig-alkoholischen Extrakt von Khaya senegalensis, vorzugsweise einem Rindenextrakt dieser Pflanze, besteht.

5. Synergistischer Komplex nach einem der Ansprüche 1 bis 4, **dadurch gekennzeichnet, daß** die vorliegende(n) Aminosäure(n) zur Gruppe Histidin, Arginin und Tyrosin gehört bzw. gehören.

6. Synergistischer Komplex nach Anspruch 5, **dadurch gekennzeichnet, daß** die Aminosäuren in Form von Bernsteinsäure- und/oder Asparaginsäuresalzen vorliegen.

7. Synergistischer Komplex nach Anspruch 5, **dadurch gekennzeichnet, daß** die Aminosäuren in reiner Form, in Form von Berns teinsäure- und/oder Asparaginsäuresalzen und/oder in Form von solchen Mischungen (Aminosäuresalz oder Aminosäure/Bernsteinsäure oder Asparaginsäure) vorliegen und gemeinsam 0,05 Gew.-% bis 40 Gew.-%, vorzugsweise 0,1 Gew.-% bis 40 Gew.-% des synergistischen Wirkkomplexes ausmachen.

8. Synergistischer Komplex nach einem der Ansprüche 1 bis 7, **dadurch gekennzeichnet, daß** er außerdem 0,05 Gew.-% bis 5 Gew.-% eines wasserlöslichen Extrakts von Hefezellen der Art Saccharomyces cerevisiae enthält.

9. Synergistischer Komplex nach einem der Ansprüche 1 bis 8, **dadurch gekennzeichnet, daß** er außerdem 0,05 Gew.-% bis 10 Gew.-% an Osen, Polyosiden und/oder Polysacchariden, insbesondere Saccharose und/oder Glycogen, enthält.

10. Synergistischer Komplex nach einem der Ansprüche 1 bis 9, **dadurch gekennzeichnet, daß** er außerdem 0,01 Gew.-% bis 10 Gew.-% B-Vitamin(e) wie inbesondere Pyridoxin und/oder Niacinamid, enthält.

11. Verwendung eines synergistischen Komplexes nach einem der Ansprüche 1 bis 10 als Wirkstoff zur Herstellung eines Kosmetikprodukts bzw. einer Kosmetikzusammensetzung und/oder eines Arzneimittels bzw. einer Arzneimittelzusammensetzung zur topischen Verwendung für die Haut und/oder die Epithelgebilde.

12. Verwendung nach Anspruch 11, **dadurch gekennzeichnet, daß** der synergistische Komplex als Wirkstoff gegen Hautstreß verwendet wird, was anhand einer Verringerung der Expression von unter der Bezeichnung HSP, insbesondere HSP27, bekannten Streßproteinen belegt wird.

13. Verwendung nach Anspruch 11 oder 12, **dadurch gekennzeichnet, daß** der synergistische Wirkkomplex als Stimulans für die Autosynthese von reduziertem Glutathion durch die Haut- oder Kapillarzellen verwendet wird.

14. Verwendung nach einem der Ansprüche 11 bis 13, **dadurch gekennzeichnet, daß** der synergistische Komplex als Mittel gegen das Auftreten von durch akute Sonnenstrahlung induzierten oder aufgrund der chronischen lichtinduzierten Hautalterung entstandenen Disulfidbrücken verwendet wird.

15. Verwendung nach einem der Ansprüche 11 bis 14, **dadurch gekennzeichnet, daß** der synergistische Wirkkomplex in ein Sonnenschutzmittel oder in ein Tagesprodukt für die Hautpflege mit einer vorbeugenden und/oder wiederaufbauenden Wirkung gegen Alterungserscheinungen eingearbeitet wird.

16. Kosmetikprodukt für die Haut und/oder die Epithelanhanggebilde mit insbesondere einer molekularen Lichtschutzwirkung auf die Zellen, **dadurch gekennzeichnet, daß** es 0,01 Gew.-% bis 90 Gew.-% eines erfindungsgemäßen synergistischen Komplexes nach einem der Ansprüche 1 bis 10 enthält.

17. Arzneimittel für die Haut und/oder die Epithelanhanggebilde mit insbesondere einer molekularen Lichtschutzwirkung auf die Zellen, **dadurch gekennzeichnet, daß** es 0,01 Gew.-% bis 90 Gew.-% eines erfindungsgemäßen synergistischen Komplexes nach einem der Ansprüche 1 bis 10 enthält.

## Claims

1. Synergetic complex, in particular to be integrated in a cosmetic and/or pharmaceutical preparation for topical use for the skin and/or superficial body growth (hair, nails, teeth etc.), **characterised in that** it comprises at least a Pisum sativum seed extract which is rich in peptides, a plant extract from the Meliaceae family which is rich in tannins and/or coumarinic derivatives and, if necessary, at least one amino-acid in the form of complex salt(s).

2. Synergetic complex according to claim 1, **characterised in that** it comprises between 0.05 and 40 wt.%, preferably between 1 and 40 wt.% of Pisum sativum extract which is rich in peptides, in dehydrated form or otherwise.

3. Synergetic complex according to any one of claims 1 and 2, **characterised in that** it comprises between 0.005 and 10 wt.%, preferably between 0.1 and 10 wt.%, of plant extract from the Meliaceae family which is rich in tannins and/or coumarinic derivatives.

4. Synergetic complex according to claim 3, **characterised in that** the plant extract from the Meliaceae family consists of a dehydrated aqueous, alcoholic or hydroalcoholic extract of Khaya senegalensis, preferably an extract of the cortex of this plant.

5. Synergetic complex according to any one of claims 1 to 4, **characterised in that** the amino-acid or the amino-acids present belong(s) to the group formed by histidine, arginine and tyrosine.

6. Synergetic complex according to claim 5, **characterised in that** the amino-acids are present in the form of succinic acid salts and/or aspartic acid.

7. Synergetic complex according to claim 5, **characterised in that** the amino-acids are present in pure form, in the form of succinic acid salts and/or aspartic acid and/or in the form of mixtures of the type (amino-acid salt or amino-acid/succinic acid or aspartic acid), together representing between 0.05 and 40 wt.%; preferably between 0.1 and 40 wt.% of the active synergetic complex.

8. Synergetic complex according to any one of claims 1 to 7, **characterised in that** it also comprises between 0.05 and 5 wt.% of a hyrdrosoluble extract of yeast cells of the Saccharomyces cerevisiae type.

9. Synergetic complex according to any one of claims 1 to 8, **characterised in that** it also comprises between 0.5 and 10 wt.% monosaccharoses, polyosides and/or polysaccharides, in particular saccharose and/or glycogene.

10. Synergetic complex according to any one of claims 1 to 9, **characterised in that** it also comprises between 0.01 and 10 wt.% of vitamin(s) of the B group, such as in particular pyridoxine and/or niacinamide.

11. Use of a synergetic complex according to any one of claims 1 to 10 as active ingredient for the preparation of a composition or a cosmetic and/or pharmaceutical product for topical use for the skin and/or superficial body growth (hair, (teeth), (nails) etc.).

12. Use according to claim 11, **characterised in that** the synergetic complex is used as a cutaneous anti-stress active agent, demonstrated by a reduction in the manifestation of stress proteins known under the name HSP, in particular HSP27.

13. Use according to any one of claims 11 and 12, **characterised in that** the active synergetic complex is used as stimulating agent for the reduced autosynthesis of glutathione by the cutaneous or capillary cells.

14. Use according to any one of claims 11 to 13, **characterised in that** the active synergetic complex is used as an agent which opposes the appearance of disulphide bridges induced by acute solar radiation or as result of photo-induced chronic cutaneous ageing.

15. Use according to any one of claims 11 to 14, **characterised in that** the active synergetic complex is integrated in a product of the solar photo-protective type or in a product for cutaneous day care, with a preventative and or restorative action for the effects of ageing.

16. Cosmetic product for the skin and/or superficial body growth (hair, (teeth), (nails) etc.), having in particular a molecular cytophoto-protective activity, **characterised in that** it comprises between 0.01 and 90 wt.% of a synergetic complex according to any one of claims 1 to 10.

17. Pharmaceutical product for the skin and/or superficial body growth (hair, (teeth), (nails) etc.), having in particular a molecular cytophoto-protective activity, **characterised in that** it comprises between 0.01 and 90 wt.% of a synergetic complex according to any one of claims 1 to 10.
